# EUROPEAN PATENT APPLICATION

(11) **EP 4 588 493 A1**
(43) Date of publication of application: **23.07.2025**
(21) Application number: 24152974.2
(22) Date of filing: 19.01.2024
(51) Int. Cl.: A61L 27/16, A61L 27/50, A61L 31/04, A61L 31/14

(54) **ENHANCE CELL-MATERIAL INTERACTION FOR INGROWTH OF EPTFE-SLEEVED CARDIOVASCULAR STENT BASED ON PHYSICOCHEMICAL SURFACE PROPERTIES**

(71) Applicant: RIJKSUNIVERSITEIT GRONINGEN, 9712 CP Groningen (NL); Academisch Ziekenhuis Groningen, 9713 GZ Groningen (NL); BiomACS BV, 9713 ED Groningen (NL)
(72) Inventor: Hamstra, Richard, 9315 PA Roderwolde (NL); Van Rijn, Patrick, 9468 GR Annen (NL); Jurczak, Klaudia, 2321 KL Leiden (NL); Schuurman, Richte, 3812 VA Amersfoort (NL); De Vries, Jean-Paul, 1405 EC Bussum (NL)
(74) Representative: Pot, Emil

(57) **Abstract**

The invention relates to the fields of biomaterials, tissue engineering and regenerative medicine. More specifically, it relates to biomaterial substrates having precise surface properties and the use thereof to enhance the attachment and ingrowth of ePTFE sleeved stents with the blood vessel wall. The specific physicochemical properties stimulate smooth muscle cell attachment and spreading that enables the stent to be biologically fixated in a very intimate fashion. This biological fixation prevents migration, infection, and aneurysm progression.

## Description

The invention relates to the fields of biomaterials, tissue engineering, and regenerative medicine. More specifically, it relates to biomaterial substrates having precise surface properties and the use thereof to direct cellular response for enhancing medical implant performance. Even more so, it relates to the enhancement of a cardiovascular stent that are ePTFE (expanded PolyTetraFluoroEthyele) sleeved, a metal stent that has an ePTFE sleeve around it to be the barrier between the metal stent and the blood vessel wall, and that is enhanced to have the blood vessel wall attach to the surface of the ePTFE sleeve.

### Background of the Invention

It is well known to use extruded tubes of polytetrafluoroethylene (PTFE) as implantable vascular stents. PTFE is particularly suitable as an implantable prosthesis as it exhibits superior biocompatibility. PTFE tubes may be used as vascular stents in the replacement or repair of a blood vessel as PTFE exhibits low thrombogenicity. In vascular applications, the stents are manufactured from expanded polytetrafluorethylene (ePTFE) tubes. These tubes have a microporous structure which allows natural tissue ingrowth and cell endothelization once implanted in the vascular system. This contributes to long term healing and patency of the stent.

Although PTFE stents are frequently used in cardiovascular applications, it may cause complications such as migration, infection, occlusion and aortic rupture (Wanhainen European Journal of Vascular and Endovascular Surgery. 2019). Stent-graft migration is a major complication for instance after placing a stent for an aortic aneurysm (Venermo. Journal of Endovascular Therapy. 2011), which increases the risk of developing endoleaks and is due to a lack of stent graft fixation. But also infections that reside between the vessel wall and the stent or in case of an aneurysm, the progressive widening of the blood vessel in other locations and the migration due to the pulsating flow that occurs within the cardiovascular system. For these complications, improvement of graft fixation will prevent and improve the patency of such stents, particularly when it entails biological fixation. Conventionally, ePFTE, chemically identical to Teflon, will not have any interaction with a biological system and no attachment to the cell wall, thus no biological fixation will occur.

To overcome the complications of using a cardiovascular stent from PTFE material, stent graft fixation needs to be improved by inducing cell attachment and proliferating vascular smooth muscle cells (SMCs). It is an essential element of the invention hereunder that the SMCs should be targeted since the endothelial cells (ECs) will be damaged/removed upon placing a stent and other cells such as fibroblasts are not immediately in direct contact, hence making SMC the most appropriate target cell for inducing cell adhesion.

It has already been suggested that stent-graft fixation can be improved by induction of vascular cell growth into the stent-grafts (van der Bas, Journal of Vascular Surgery. 2004) by creating a Dacron stent-graft impregnated with collagen, heparin, and fibroblast growth factor (FGF) and demonstrated *in vivo* stent-graft incorporation and healing between the graft and the aorta. The study remained experimental but proved successful, however one would like to avoid the addition of such biological factors as it is considered a device combined with pharmaceutically active compounds (drugs).

It has also been shown that vascular endothelial cells, smooth muscle cells, lens epithelial cells, fibroblasts, osteoblasts, and mesenchymal stem cells respond to physicochemical properties such as topography, wettability, and stiffness, and the combinations thereof, and alter their phenotype. (Han, Biochemical and Biophysical Research Communications. 2020)(Almonacid Suarez, Heliyon. 2020)(Zhou, Adv. Mater. Interfaces, 2018)(Zhou, Adv. Mater. Interfaces, 2016)(Kuhn, ChemNanoMat, 2016).

### Description of the Invention

Despite that it has been shown that cell growth depends on physicochemical properties of the surface of vascular stents materials, it is not obvious to determine what such optimal physicochemical properties should be for each specific surface material in combination with a specific cell type. For the polymeric ePTFE-film lining of stent-grafts that are currently being used clinically we have tested numerous different physicochemical properties with the growth of SMCs as a primary endpoint.

Surprisingly, we found that a vascular stent comprising of ePTFE surface material characterized by a surface oxygen content between 1.8-2.8% and having a water contact angle between 110 - 125 degrees displayed the most favorable enhanced cell adhesion and cell proliferation, in particular of vascular SMCs. These were shown to be 5.9 times higher on the modified stent surfaces than on the non-modified stent surfaces.

Thus, the invention hereunder provides for a vascular stent comprising of ePTFE surface material characterized by a surface oxygen content between 1.8-2.8% and having a water contact angle between 110 - 125 degrees.

An important embodiment of the vascular stent of the invention is for use as an implant in a blood vessel, resulting in a stronger connection between the stent graft and the vessel wall, reflected in an enlarged neointima-stent surface interaction that results in a higher force to separate the stent from the tissue. Another important embodiment of the vascular stent of the invention is that said increased interaction would ultimately translate into less movement of the stent and prevention of the aneurysm progression and as such will improve clinical outcomes.

The invention hereunder also provides for a manufacturing process of a vascular stent comprising of ePTFE surface material characterized by a surface oxygen content between 1.8-2.8% and having a water contact angle between 110 - 125 degrees by applying air-plasma flow at the surface of a stent of ePTFE material during 35 seconds at a pressure 100mTorr and with 100% intensity of 40kHz / 200 Watt power.

### EXAMPLES:

### Example 1:

### 1.1. Generation of the Water Contact Angle Gradient on ePTFE using Oxygen Plasma and Gradient Characterization

### 1.1.1 Generation of the Water Contact Angle Gradient on ePTFE using Oxygen Plasma

To produce wettability gradients on ePTFE (Bentley InnoMed), ePTFE was cut into 3,0 × 1,5 cm samples and sticked to a glass slide with a double tape. A triangular 30° angular prism mask was placed on top of the ePTFE sample and the position of the closed side of the prism mask was marked on the sample. Then, 5 positions (0,1 cm; 0,5 cm; 1,0 cm; 1,5 cm and 1,9 cm) along the length of the prism mask were marked on the edge of the sample. The sample was oxidized in air plasma under a pressure of 100 mTorr for 60 sec air plasma with 100% intensity of 40kHz / 200 Watt using a Diener Atto plasma setup. Prior the treatment the pressure at 100 mTorr is stabilized for approximately 60sec.

### 1.1.2. Contact angle characterization

The contact angle measurements were performed to determine the wettability of the ePTFE sample along the formed wettability gradient using in-house build contact angle measuring device. An ultrapure water (Milli-Q) droplet was placed on the surface of the sample, for 1 gradient point the 3 independent measurements were taken.

### 1.1.3. X-ray photoelectron spectroscopy analysis.

In order to understand the influence of the plasma treatment on the degree of oxidation along the gradient, we investigated the surface chemistry of all the points of the gradient. Chemical composition of all the surfaces was characterized by X-ray photoelectron spectroscopy (XPS). XPS was conducted using an SSI S-Probe (Surface Science instrument, Mountain View, CA, USA) with a monochromatic Al X-ray source (1486.8 eV). The pass energy is set at 150 eV for the survey spectra) and to 50 eV for the detailed spectra of the C 1*s*, O1s and F1s core level regions. The spectra has been processed using the CasaXPS software. In a single experiment, atomic compositions of all the points along the gradient surfaces were quantified sequentially, where each gradient point surface was scanned three independent times and the results of the three scans were averaged. The same experiment was repeated three independent times. Binding energies are reported ±0.1 eV and referenced to the peak centered at a binding energy of 284.8 eV. of the spectra included a Shirley baseline subtraction and fitting with a minimum number of peaks consistent with the chemical structure of the sample, taking into account the experimental resolution. The profile of the peaks was taken as a convolution of Gaussian and Lorentzian functions. The uncertainty in the peak intensity determination is 2% for all core levels reported.

### 1.1.4 Scanning Electron Microscopy

The morphology of ePTFE stents was imaged via scanning electron microscopy (SEM). Prior imaging, the samples were sputtered with 10 nm Pd/Au using Leica EM SCD050 sputtercoater and observed with Atlas Zeiss field emission SEM operating at 3kV.

### Example 2

### 1.2. Isolation of Smooth Muscle Cells from the Porcine Origin

### 1.2.1 Smooth Muscle Cells Isolation from the Porcine Origin

Arteries were obtained from 6-month-old female Yorkshire pigs from the local slaughterhouse (Kroon Vlees, Groningen, The Netherlands). All pigs were slaughtered according to the laws and principles of the Dutch Food and Consumer Product Safety Authority. Upon receiving the fresh aorta, the tissue was immediately placed in cold PBS supplemented with 1% penicillin/streptomycin (Gibco) and 1% amphotericin B (Sigma Aldrich). The arterial tissue was transported to the laboratory in a polystyrene cooling box filled with ice. Fresh porcine aorta, 10 to 14 cm in length was rinsed with cold calcium and magnesium free phosphate-buffered saline (PBS) supplemented with 1% penicillin/streptomycin (Gibco), and 2.5 pg/mL of amphotericin B (Sigma Aldrich) to remove all residual blood. The connective tissue and adventitia were removed using forceps and small spring scissors on 150 mm dish. The aorta was cut open longitudinally using the small spring scissors and spread open so that the intima was faced up. Using the cell scraper the endothelial layer was removed from the aorta. The tissue was cut into 1-2 mm pieces, transferred to 50ml falcon tubes and incubated for 1.5 hours in 0.2% (w/v) collagenase type III (Stemcell Technologies) in DMEM F12 (Gibco) in the room temperature on a shaking plate. The collagenase solution was gently removed, discarded and tissue was placed on 4-5cm petri dishes. Tissues were covered with 0.1% (w/v) collagenase type III (Stemcell Technologies) in DMEM F12 (Gibco and incubated for **8-10 hours** at 37°C. The cell-enzyme solution was deactivated with equal volume of proliferation medium consisted of Dulbecco's modified Eagle's medium (DMEM) with 4,5 g/L D-Glucose (Gibco) that was supplemented with 20% fetal bovine serum (FBS) (Thermo Scientific), 1% penicillin/streptomycin (Gibco), 0,1 % human epidermal growth factor (hEGF, Peprotech), 0,1% insulin (Sigma Aldrich), 0,2% basic fibroblast growth factor (bFGF, Peprotech) and 1% amphotericin B (Sigma Aldrich). The cell-enzyme solution was filtered through a 70 *µ*m - cell strainer to eliminate large pieces of aortic tissue. The collected cell suspension was centrifugated at 1500 rpm for 15 min in a room temperature. The cell pellet was resuspended and cultured in proliferation medium at 37°C with 5% CO₂. The medium was added to the flask on day 2, cell were left for 5-7 days and passaged when reached 80% confluency.

### 1.2.2 Flow Cytometry Characterization

Aortic tissue consists of three layers of cells i.e. endothelial cells, smooth muscle cells and fibroblasts. The cell sorting was performed to yield the higher concentration of smooth muscle cells, which were denoted as cells positive for smooth muscle actin and myosin heavy chain 11, and negative for CD31 marker. All stainings were done on ice with the minimal exposure to light. Cells were treated with Trypsin/EDTA to detach them from the cell flask and later centrifuged at 1800 rpm for 5 min. Cells were washed with PBS and fixed with 1 ml of 3,7 % warm Paraformaldehyde (PFA) for 15min. The 0,1 % Triton X-100 was used for the cell permeabilization and non-specific binding was blocked using 5% BSA in PBS. The staining was done using the following antibodies: Mouse Anti-Rat CD31 conjugated with BD Horizon^{™} BB515 (BDBioscience, 10ug/ml), Myosin Heavy Chain 11cnjugated with Allophycocyanin (Novus Biologicals, 20ug/ml)), alpha smooth muscle actin conjugated with Phycoerythrin (Novus Biologicals, 20ug/ml). Antibodies were added to 100 ul of cell suspension in low-binding 1.7 mL vials (Cat. # 3207, Costar) and were incubated for one 30 min at RT. Afterwards, cells were centrifuged for 5 min at 1800 rpm and washed 2 times with 1% PBS. The pellet was resuspended in 100 uL 1% BSA in PBS to proceed with flow cytometry within 30 min. The samples were transported at RT, and flow cytometry experiments were performed in MoFlo Astrois Cell Sorter cytometer (Beckman Coulter). The population of cells that was positive for a-SMA and MYH11 was put back to culture in conditioned medium and used for the further experiments.

### Example 3

### 1.3 Cell Culture with ePTFE Wettability Gradient

### 1.3.1. ePTFE gradients preparation for the cell culture

To produce ePTFE gradient samples for the cell culture, first the sticky layer of PDMS (SYLGARD 184 kit, Dow Corning, MI, USA) with crosslinker in a ratio 10:1 was prepared. In short, the mixture was stirred with a spatula and left to degas in a room temperature. Subsequently, the mixture was deposited on planar polystyrene petri dish and placed in the oven at 70°. The mixture was taken out from the oven once it became sticky afterwards the ePTFE samples with gradient were placed on a cooled sticky PDMS layer. After 24h the circular cutter was used to cut out the samples fitting the size for 12 well plate. Prior cell culture the samples were immersed in 70% EtOH for sterilization and washed 3x with phosphate buffer saline (PBS).

### 1.3.2. Cell Culture

Cells between passages 4 and 6 were seeded in culture plates (12 well plates) at 5000 cells/well seeding density for cell behavior screening. Cells were cultured in Dulbecco's modified Eagle's medium (DMEM) with 4,5 g/L D-Glucose (Gibco) that was supplemented with 20% fetal bovine serum (FBS) (Thermo Scientific), 1% penicillin/streptomycin (Gibco), 0,1 % human epidermal growth factor (hEGF, Peprotech), 0,1% insulin (Sigma Aldrich), 0,2% basic fibroblast growth factor (bFGF, Peprotech) and 0,1% amphotericin B (Sigma Aldrich) at 37°C in a humidified incubator with 5% CO2. The media was refreshed every 2 days. The ePTFE non-treated with plasma and tissue culture plastic (TCP) served as a control.

### 1.3.3 Immunofluorescence

At the end of cell culturing time points i.e. 24h and 5 days, the cells on ePTFE and TCP were fixed in 3.7% paraformaldehyde (Sigma) for 15 min and subsequently permeabilized with 0.5% Triton X-100 (Sigma) for 3 min. Subsequently, the unspecific binding was blocked with 5% bovine serum albumin (Sigma) in PBS solution for 30 min. Samples were incubated with primary and secondary antibody solution for 1h. The primary antibodies used were: mouse anti-human smooth muscle actin (a-SMA) antibody (A2547 Sigma Aldrich, 1:100); rabbit anti-mouse ki-67 antibody (Abcam ab15580, 1200). The secondary antibody was mouse IgG Highly Cross-Adsorbed Secondary Antibody (Thermo Fisher A32766; 1:1000) conjugated with Alexa Fluor^{™} Plus 48 and Alexa647 labeled donkey- anti-rabbit antibody (Jackson Immunolab 711605152, 1:100). Nuclei and fiber actin were stained with DAPI (Invitrogen, 1:50) and FITC/TRITC-phalloidin Atto565 (Sigma Aldrich 94072, 1:50), respectively, by incubation for 1 h. The cells were imaged with TissueFaxs, with a Zeiss AxioImager Z1Microscope System (Tissue-Gnostics GmbH, Vienna, Austria) at 10× magnification. The cell data was analysed by Tissue Quest software (high-throughput analysis technique) by creating 10 region of interest (ROI) along the gradient. The expression of a-SMA was evaluated quantitatively by determining the integrated density value, the cell area was calculated via phalloidin expression and the proliferation of the cells was evaluated by calculating number of cells expressing ki-67 and then dividing them by the total cell number, determined by nucleus staining with DAPI. The quantitative analysis was done via the TissueQuest software (high-throughput analysis technique).

### Example 4

### 1.4 Translation Study

### 1.4.1. Testing Parameters to Recreate region of interest to the ePTFE

The cell screening study enabled to determine the favorable position on the gradient for the cell adhesion, proliferation and spread growth. In order to recreate ROI we treated samples under stable pressure of 100mTorr and were changing the time duration of the air plasma with 100% intensity of 40kHz / 200 Watt power of a Diener Atto plasma equipment. The time tested was 20sec, 30 sec, 45 sec, 60 sec, 90 sec. To determine the most favorable time duration of the oxygen plasma we compared the three types of ePTFE from the same company (Bently InnoMed) and tested 30 sec, 35sec, 40 sec, 45 sec. The water contact angle measurements were performed in three independent experiments (for each sample WCA was measured three times) and the results of the three measurements were averaged. For three types of ePTFE treatment of 35 sec showed the lowest water contact angle measurement. For the further translational experiments on ePTFE samples, air plasma treatment of 35 sec under 100mTorr pressure was applied.

### 1.4.2. Stent Expansion Influence

To continue the translation study, the influence of balloon expansion on stent graft modification was tested. Prior the expansion, stent grafts (BeGraft aortic, Bentley InnoMed) dedicated for the plasma modification were placed on the in-house 3d printed inserts and treated under 100mTorr pressure for 35 sec.

The aortic tissues (detailed information on tissue harvesting is available in section 1.2.1) were placed on the PDMS sheet and tied with needles for the attachment and support during balloon pumping. A catheter with 8 mm × 8 cm balloon (Armada 35, Abbott) was inserted inside the tissue. The balloon was inflated (Encore^{™} 26 inflator, Boston Scientific, Marlborough, MA, USA) to a pressure of 8 atm compliant with the vendor instructions for use. After 3 min, the balloon was deflated and gently withdrawn from the tissue. Stent grafts were cut longitudinally, metal stent was separated from the ePTFE graft material. The ePTFE material was prepared for the cell culture as discussed in section 1.3.1.

The study compared *non- expanded* ePTFE stent grafts treated with plasma (time=35 sec; pressure=100mTorr; sample 1) and not-treated with plasma (control 1) as well as *expanded* ePTFE stent grafts treated with plasma (time=35 sec; pressure=100mTorr; sample 2) and not-treated with plasma (control 2). Cells at passage 7 were seeded in culture plates (96 well plate) at 2000 cells/ well seeding density and cultured in Dulbecco's modified Eagle's medium (DMEM) with 4,5 g/L D-Glucose (Gibco) that was supplemented with 20% fetal bovine serum (FBS) (Thermo Scientific), 1% penicillin/streptomycin (Gibco), 0,1 % human epidermal growth factor (hEGF, Peprotech), 0,1% insulin (Sigma Aldrich), 0,2% basic fibroblast growth factor (bFGF, Peprotech) and 0,1% amphotericin B (Sigma Aldrich) at 37°C in a humidified incubator with 5% CO2. The expansion influence was investigated after 24h and 5 days cell growth on the stent graft material.

Based on the images and quantified by assessing the biomass on the stent surface (based on phalloidin output), showing a 5.9-fold increase in biomass, it is envisioned that the modification is sufficiently inducing SMC growth and stimulation to serve the purpose of improved stent graft attachment.

### 1.4.3. Fabrication influence on stent graft modification.

This study was conducted to trace whether the plasma modified stent grafts would withstand the fabrication process of placing stent graft onto the balloon catheter. First the stent grafts were delivered to University Medical Centre Groningen and were plasma modified as described in the section 1.4.2. During each plasma treatment a control ePTFE material was placed together with stents inside the chamber to control the change in the water contact angle as described in section 1.1.2. The modified stents were delivered to Bentley InnoMed (Hechingen, Germany) where they underwent the fabrication steps i.e. crimping (Fab1), crimping and embedding (Fab2), crimping, embedding and packaging (Fab3) and crimping, embedding, packaging and sterilization (Fab4). All the steps were conducted in a GMP environment in compliance with the company's protocols.

After fabrication, stents were expanded (1.4.2.) and prepared for the cell culture. Cells at passage 6 to 9 were seeded in culture plates (96 well plate) at 4000 cells/ well seeding density and cultured in Dulbecco's modified Eagle's medium (DMEM) with 4,5 g/L D-Glucose (Gibco) that was supplemented with 20% fetal bovine serum (FBS) (Thermo Scientific), 1% penicillin/streptomycin (Gibco), 0,1 % human epidermal growth factor (hEGF, Peprotech), 0,1% insulin (Sigma Aldrich), 0,2% basic fibroblast growth factor (bFGF, Peprotech) and 0,1% amphotericin B (Sigma Aldrich) at 37°C in a humidified incubator with 5% CO2. The fabrication test was investigated after 24h and 5 days cell growth on the stent graft material. Stents that were plasma modified but did not undergo fabrication process (Fab0), stents that did not go through fabrication steps and were modified right before the cell culturing (Fab0C) and non-modified but commercially available stent grafts (NT) served as controls to the study. The cells were stained according to the protocol presented in section 1.3.3.

### 1.4.4. Ageing influence on stent graft modification.

This study was conducted to trace the influence of the shelf-life on the plasma modified stent grafts. First the stent grafts were delivered to University Medical Centre Groningen and were plasma modified as described in the section 1.4.2. During each plasma treatment a control ePTFE material was placed together with stents inside the chamber to control the change in the water contact angle as described in section 1.1.2. The modified stents were delivered to Bentley InnoMed (Hechingen, Germany) where they underwent the accelerated ageing process (in 55°C) for 1 month (Age1) that was representing real-time storage of 0.45 years and 3 months (Age3) that was representing the real-time storage of 1.39 years. All the steps were conducted in a GMP environment in compliance with the company's protocols.

After ageing, stents were expanded (1.4.2.) and prepared for the cell culture. Cells at passage 6 to 9 were seeded in culture plates (96 well plate) at 4000 cells/ well seeding density and cultured in Dulbecco's modified Eagle's medium (DMEM) with 4,5 g/L D-Glucose (Gibco) that was supplemented with 20% fetal bovine serum (FBS) (Thermo Scientific), 1% penicillin/streptomycin (Gibco), 0,1 % human epidermal growth factor (hEGF, Peprotech), 0,1% insulin (Sigma Aldrich), 0,2% basic fibroblast growth factor (bFGF, Peprotech) and 0,1% amphotericin B (Sigma Aldrich) at 37°C in a humidified incubator with 5% CO2. The ageing influence on cell-ePTFE interaction was investigated after 24h and 5 days cell growth. Stents that were plasma modified but did not go through accelerated ageing (Age0), and non-modified but commercially available stent grafts (NT) served as controls to the study. The cells were stained according to the protocol presented in section 1.3.3.

### LEGEND TO THE FIGURES

Figure A - Comparison of currently used ePTFE (left) and modified ePTFE (right) for the attachment and growth of smooth muscle cells. Left are the fluorescence images of the cells at the surface and on the right the quantification of the amount of phalloidin.

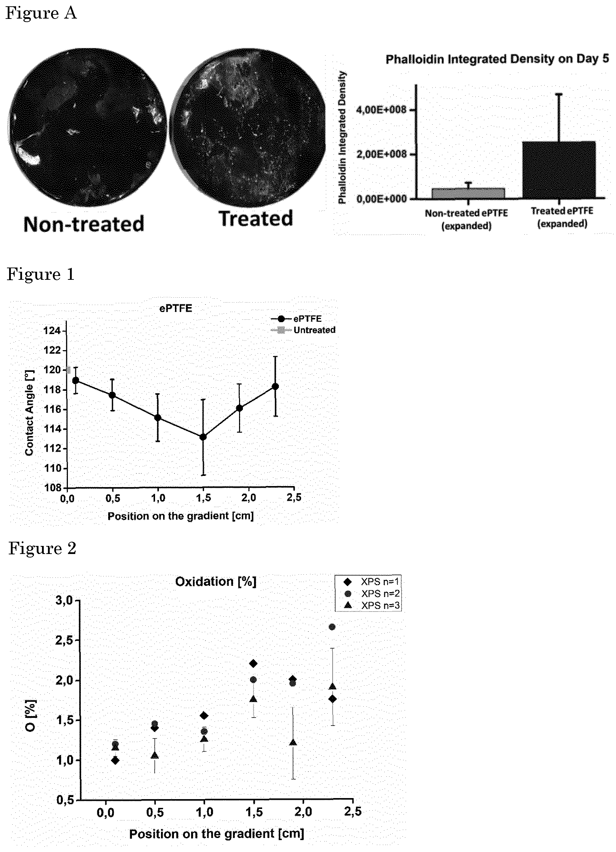

Figure 1 - Wettability gradient for ePTFE when plasma oxygen treatment of 1 min 100mTorr is applied.
Figure 2 - Degree of the oxidation on the ePTFE wettability gradient determined by X-ray photoelectron spectroscopy. Each point represents the average % from two measurements.

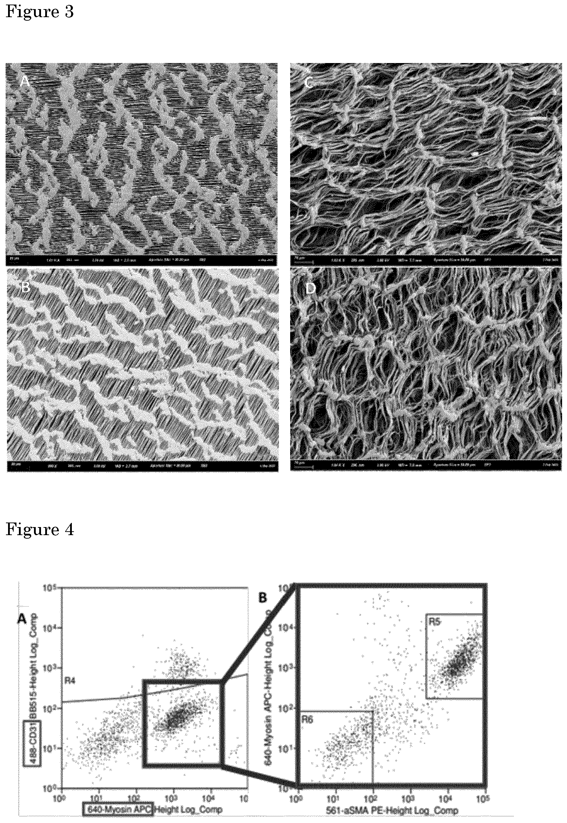

Figure 3 - Scanning electron microscopy (SEM) images of ePTFE from Bentley InnoMed non-modifed (A,C) ands plasma modified 1min_100mTorr (B,D). ePTFE that was used for cell screening experiments (A,B) and ePTFE taken for translational study (C,D).
Figure 4 - Cell sorting of isolated cells. In the first sorting step population of cells negative for CD31 and positive for Myosin marker was detected (A). In the next step the double positive population for a-SMA and mature smooth muscle marker - Myosin (R5) was identified and cultured for the experiments (B).

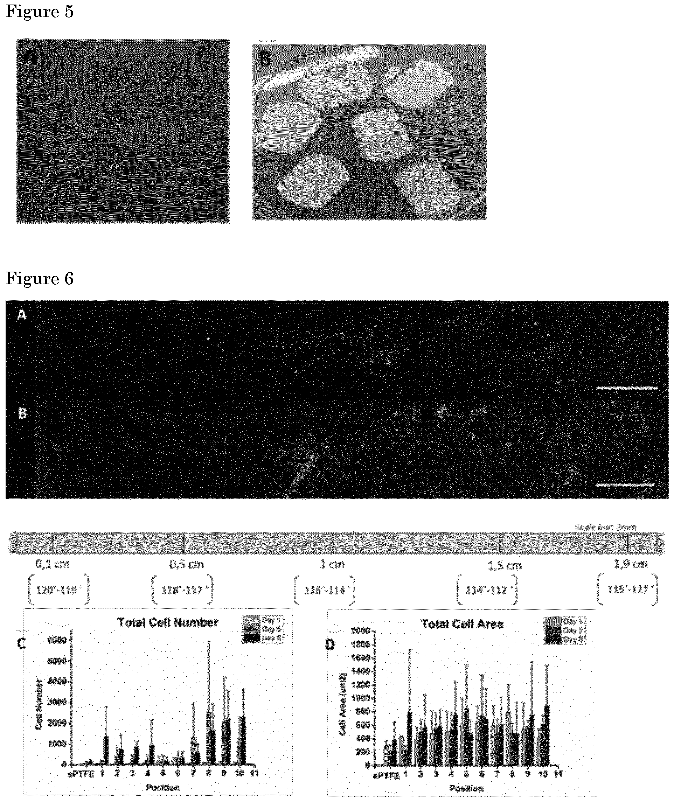

Figure 5 - ePTFE preparation for screening study. Creation of the gradient in the oxygen plasma chamber (A). ePTFE samples glued to the PDMS and prepared for the cell culture (B).
Figure 6 - Immunodetection of phalloidin (in red) and nucleus staining (in blue) of cells cultured on ePTFE wettability gradients for A) 1 and B) 5 days Scale bar represents 2 mm for the gradients. Data are shown as mean ± standard deviation (SD) for C) cell number and D) cell area. For each gradient point, there are two analysis points i.e for point 1 on a gradient, cell analysis data (C, D) is represented by point 1 (starting position) and point 2 (closer to point 2 on a gradient). Each experiment was performed in triplicate.

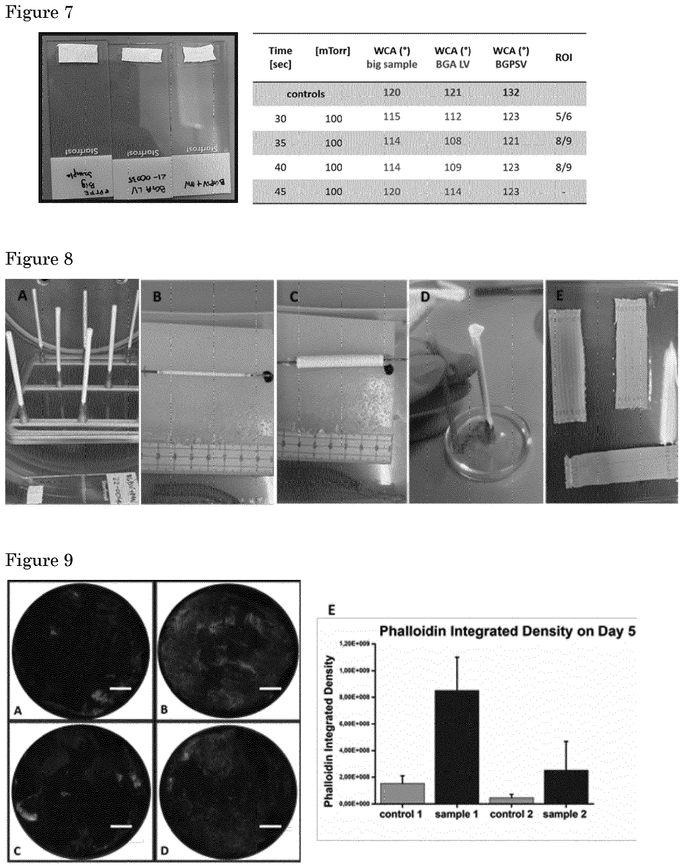

Figure 7 - Three types of ePTFE from Bentley InnoMed were investigated under various time conditions in oxygen plasma chamber. For three type of ePTFE treatment of 35 sec showed the lowest water contact angle measurement represented in Fig6D as point 8 i.e water contact angle 114°.
Figure 8 - Workflow for the stent graft expansion procedure. A)Placement of the stent grafts inside the oxygen plasma and their treatment; B) Mounting the stent to limits its movement and inserting catheter with the balloon; C) Pumping the balloon; D) Detaching metal stent from the ePTFE graft material; E) Preparing ePTFE grafts for the cell culturing experiments.
Figure 9 - Fluorescence images representing the expansion influence study. The study compares non-treated and non-expanded ePTFE (A - control1); treated and non-expanded ePTFE (B- sample 1); non-treated expanded ePTFE (C- control2) and treated and expanded ePTFE (D- sample2). Quantification of the biomass on the stent surface via phalloidin (E).

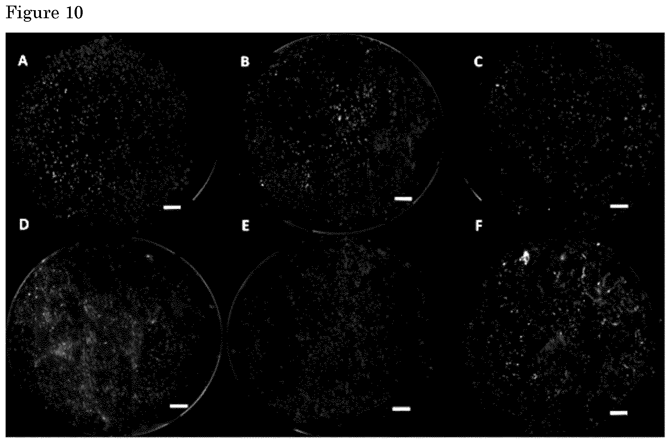

Figure 10 - Immunodetection of phalloidin (in red), nucleus staining (in blue) and alpha-smooth muscle actin (green) of cells cultured on ePTFE fabrication samples for A-C) land D-F) 5 days. Samples that were plasma modified but did not go through the fabrication process (A,D - Fab0), samples modified and fully fabricated (B,E - Fab4) and non-modified commercially available stent (C,F - NT). Scale bar represents 500 um. Magnification 10x.

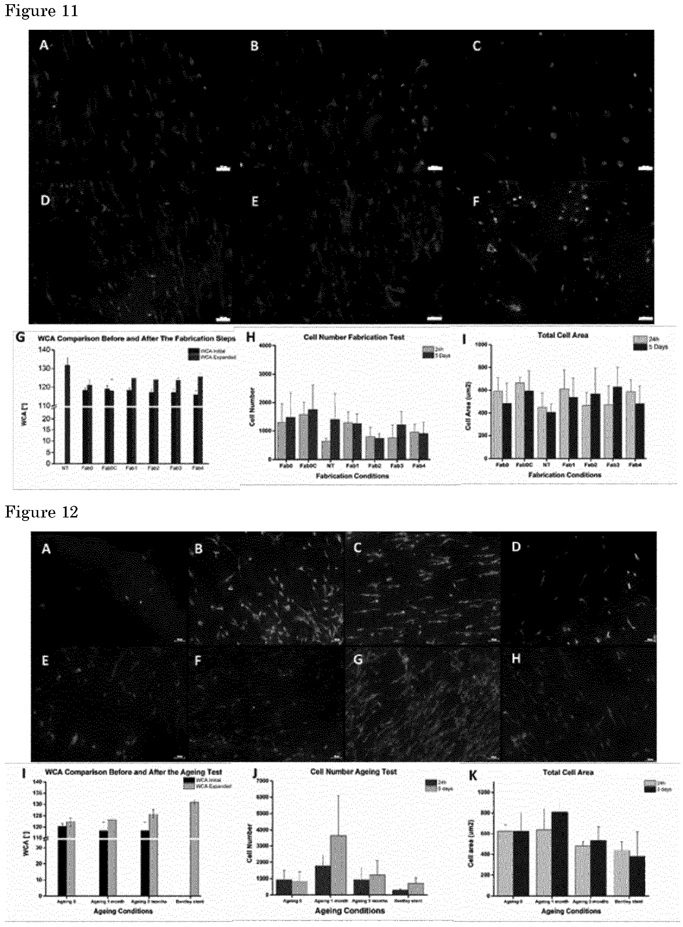

Figure 11 - Zoomed-in Immunodetection of phalloidin (in red), nucleus staining (in blue) and alpha-smooth muscle actin (green) of cells cultured on ePTFE fabrication samples for A-C) 1 and D-F) 5 days. Samples that were plasma modified but did not go through the fabrication process (A,D - Fab0), samples modified and fully fabricated (B,E - Fab4) and non-modified commercially available stent (C,F - NT). Scale bar represents 100 um. Comparison of the water contact angle before fabrication test and after (G); total cell number calculated via nuclei staining (H) and cell area determined via phalloidin staining (I).
Figure 12 - Immunodetection of phalloidin (in red), nucleus staining (in blue) and alpha-smooth muscle actin (green) of cells cultured on ePTFE ageing samples for A-D) 1 and E-H) 5 days. Samples that were plasma modified but were not aged (A,E - Age0), samples aged for 1 month (B,F - Age 1), samples aged for 3 months (C, G - Age3) and non-modified commercially available stent (D,H - NT). Scale bar represents 100 um. Comparison of the water contact angle before ageing test and after (I); total cell number calculated via nuclei staining (J) and cell area determined via phalloidin staining (K).

## Claims

1. A vascular stent comprising of ePTFE surface material **characterized by** a surface oxygen content between 1.8-2.8% and having a water contact angle between 110 - 125 degrees.

2. A vascular stent according to claim 1, for use as an implant in a blood vessel.

3. A vascular stent according to any of the claims 1 and 2, for obtaining enhanced cell adhesion and cell proliferation of vascular smooth muscles cells.

4. Manufacturing of the vascular stent according to claim 1, by applying air-plasma flow at the surface of a stent of ePTFE material during 35 seconds at a pressure 100mTorr and with 100% intensity of 40kHz / 200 Watt power.
